# EUROPEAN PATENT SPECIFICATION

(11) **EP 2 117 495 B1**
(45) Date of publication and mention of the grant of the patent: **16.05.2012**
(21) Application number: 08712650.4
(22) Date of filing: 24.01.2008
(51) Int. Cl.: A61K 6/10, A61C 9/00

(54) **DENTAL IMPRESSION MATERIAL COMPRISING AN ELASTOMER AND FERROMAGNETIC COMPOUND**
ZAHNABDRUCKMATERIAL MIT FERROMAGNETISCHEN KÖRPERN
MATERIAU D'EMPREINTE DENTAIRE COMPRENANT UN ELASTOMERE ET UN COMPOSE FERROMAGNETIQUE

(30) Priority: 01.02.2007 US 887617 P
(43) Date of publication of application: 18.11.2009
(73) Proprietor: Sefidroodi, Gholamreza, 4316 Sandnes (NO)
(72) Inventor: Sefidroodi, Gholamreza, 4316 Sandnes (NO)
(74) Representative: Onsagers AS
(86) International application number: PCT/NO2008/000023
(87) International publication number: WO 2008/094045

(56) References cited:
- DE-A1-102006 056 983
- US-A1- 2004 002 665
- DATABASE WPI Week 198611 Thomson Scientific, London, GB; AN 1986-074724 XP002509296 -& SU 1 175 465 A (VOLG MED INST) 30 August 1985 (1985-08-30)

## Description

### FIELD OF THE INVENTION

The present invention relates to elastomeric compounds used for making impressions for the fabrication of dental prostheses, more specifically to an improved compound for making dental impressions.

### BACKGROUND OF THE INVENTION

In order to fabricate a dental prosthesis such as crowns and bridges, a mold of the teeth must first be prepared. Such a mold is prepared by making an impression of the teeth using a curable molding compound applied to the teeth by dental personal or dentist. In making such a mold, it is necessary that the impression also include the sub-gingival margins of the prepared tooth. This procedure is difficult, however, because of the small space between the tooth's surface and the gingival tissue.

Previous methods for introduction of the molding compound into the sub-gingival area involved the widening of the sulcus, i.e. the sulcus and/or gingival tissue was retracted and the neck of the tooth exposed. One common method involves the insertion of a retraction cord into the sulcus. Other methods include the use of an expandable compound that causes a separation of the gingival tissue or an astringent or other substance causing tissue retraction, as well as surgical procedures for exposing the sub-gingival areas of the tooth.

Document US 2004/002665 discloses the use of a magnetorheological dental impression compound. In this document, a bite bag comprising a flexible bladder made of a thin elastomeric bag such as grade silicone is filled with a rheological material or a fluid evacuation rheological material, preferably a MR (magnetorheological) fluid. A magnet is then placed in proximity to the bite bag to activate the MR fluid to change state and to solidify.

Document SU 1175465 discloses an electromagnetic fluid or liquid.

Document DE 10 2006 056 983 discloses thermoplastic materials which are used in the dental field. These materials can be solidified by increase of the temperature, either by microwaves or by the action of a magnetic field on magnetic particles.

Neither of these prior art documents discloses an elastomeric dental impression compound to which a ferromagnetic compound has been added.

Most of the aforementioned methods involve substantial discomfort for the patient and potential complications. Furthermore, even when the gingival tissue is so widened, it has proven difficult to satisfactorily introduce the molding compound into the sub-gingival pockets in order to achieve a good impression of the subgingival margins of the tooth. A need therefore exists for a dental impression compound that can be used to make acceptable cast of the tooth and the sub-gingival areas regardless of the degree of gingival-widening or without gingival-widening, and that can be more effectively introduced into the sub-gingival areas.

### SUMMARY OF THE INVENTION

The current invention addresses the above-described disadvantages of the prior art by providing a curable dental impression compound according to claim1 comprising a ferromagnetic additive, as well as a method according to claim 10 and for guiding the compound into the sub-gingival areas of the tooth by the use of a magnetic field. The term "ferromagnetic" as used herein is used to describe a substance that is capable of being attracted by a magnetic field, in particular a substance that exhibits a high degree of magnetic permeability.

### BRIEF DESCRIPTION OF THE DRAWINGS

The invention will be explained in more detail with reference to the attached figures, wherein:
Fig 1 is a cross sectional view of a tooth prepared for the taking of an impression
Fig 2 is a cross sectional view from fig 1, showing an amount of impression compound placed at the edge of the gingival tissue
Figs 3 and 4 illustrates the use of a magnet device for guiding the impression compound downward into the gingival pocket.
Fig 5 illustrates an alternate method of using a magnet to guide impression compound into the gingival pocket

### DETAILED DESCRIPTION OF THE INVENTION

One aspect of the present invention provides for an elastomeric compound for the taking of dental impression otherwise known in the art, to which is added a ferromagnetic compound such as iron powder or carbonyl iron powder in sufficient quantity such that the compound may be physically moved by a magnetic field. Another aspect of the invention provides for a method of using a magnet to guide the aforementioned compound into the sub-gingival pockets.

### Impression compound

According to a preferred embodiment, the impression compound according to the invention comprises a light body/flowing addition polymerized silicone impression material commercially available. Carbonyl Iron powder, for example produced by BASF, Germany, is added to the impression compound in sufficient quantity as to provide the compound with the sufficient ferromagnetic property, but without negatively effecting the elasticity of the impression compound to a significant degree. The ferromagnetic property of the compound is preferably such that a magnetic field having a Tesla value within the range approved for human exposure will physically attract the compound, preferably a Tesla value of less than 2.

According to one aspect of the invention the particle size of the ferromagnetic particles of the powder is between 0.3µm - 20 µm, more preferably between 0.5 µm - 5 µm, and most preferably between 0.8 µm - 2 µm. The powder preferably represents 10%-75% by weight of the compound, more preferably between 15-55% by weight and most preferably between 20%-30% by weight.

### Method

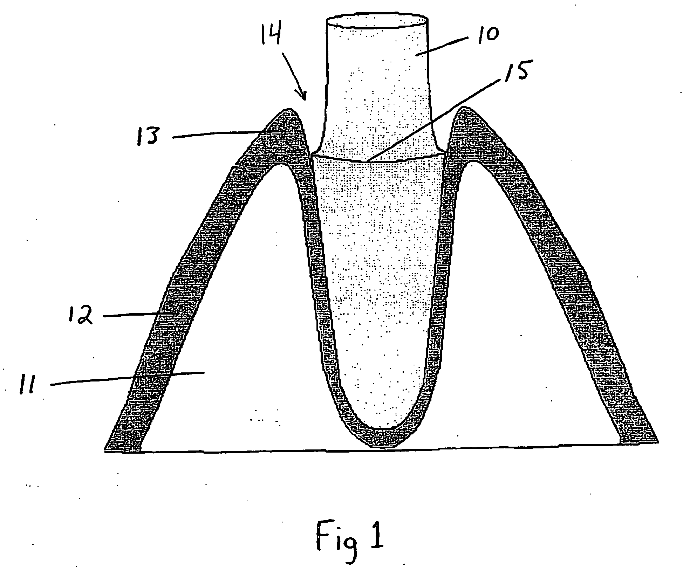

As shown in Fig 1 a tooth 10 is first prepared for the dental prosthesis (not shown). The relevant anatomical features of the tooth for the purposes of the following description include alveolar bone 11, the attached gingival tissue 12, the free gingival tissue 13, the gingival pockets 14 and the sub-gingival margins 15.

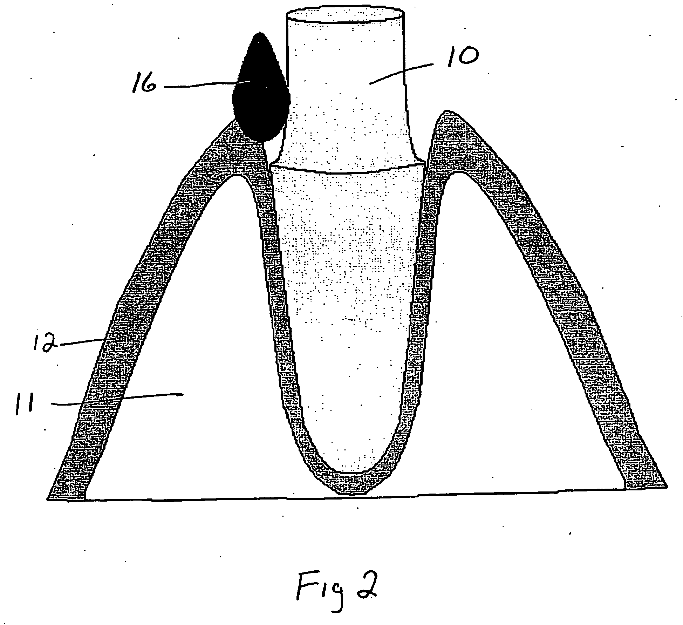

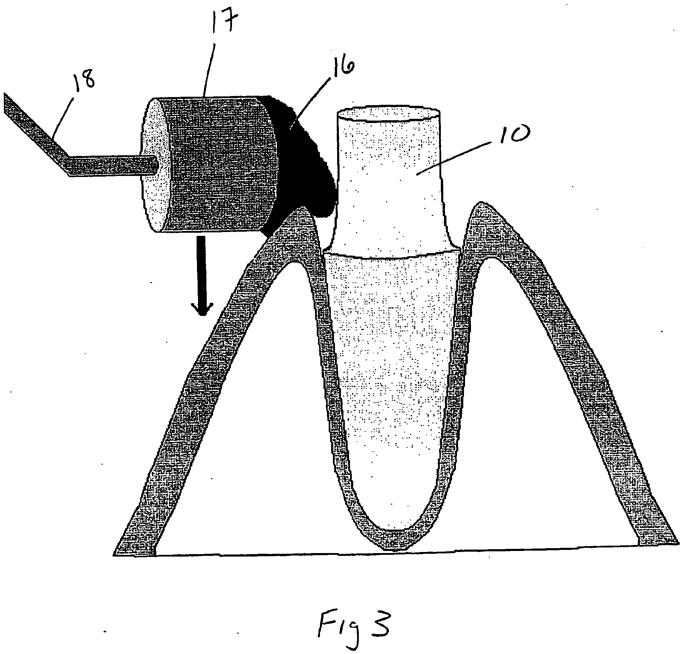

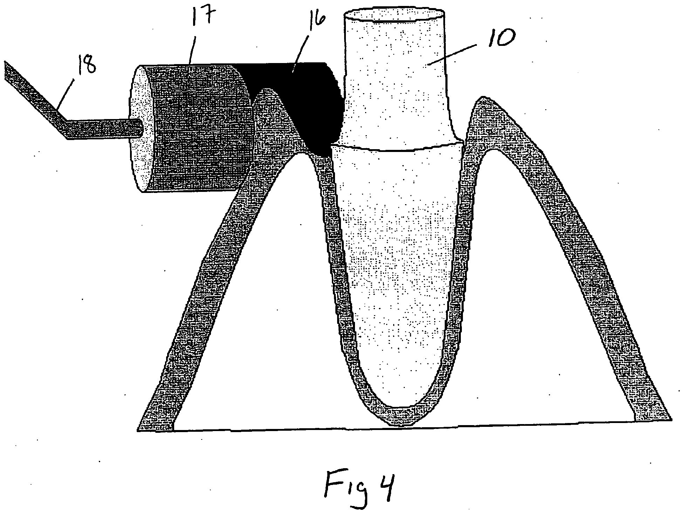

After the tooth is prepared, a small amount of impression compound 16 according to the first aspect of the invention is applied around tooth 10 at the edge of the free gingival tissue 13 as shown in Fig 2. As shown in figs 3 and 4, a magnet 17, preferably attached to a handle 18, is thereafter moved vertically against the gingival tissue from above the gum line to below the border between tooth and alveolar bone 11, thus drawing the compound down into the gingival pockets 14. After the pockets have been filled, the remainder of the compound necessary to complete the mold is applied to the tooth, and the compound polymerized according to techniques known in the art. In a preferred embodiment the magnet that is used is a cylindrical Neodymium Iron boron magnet, approximately 1 cm long and 0.5 cm in diameter, attached to a handle 18. One or several stronger or weaker magnet may also be employed, so long as the magnet exhibits a magnetic field of a strength approved for the use on humans.

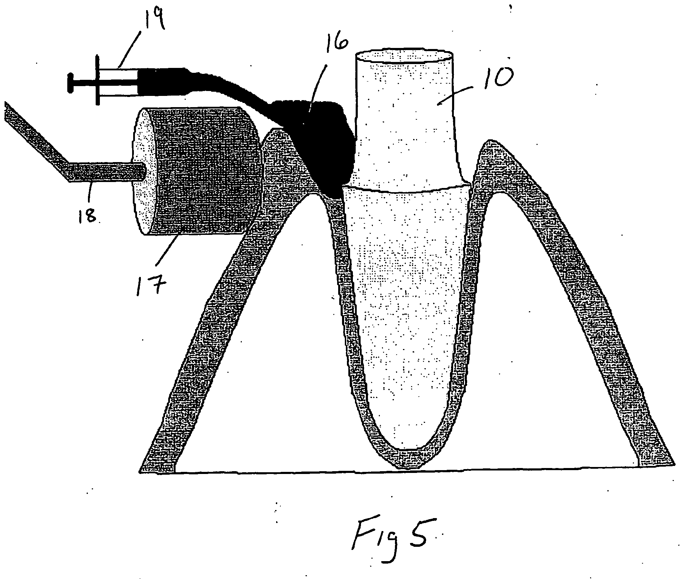

In an alternate embodiment shown in Fig 5, a syringe 19 is used to inject the impression compound 16 under the gum line while the magnet 17 is held in place below the level of the sub-gingival preparing margins 15 of the tooth. The magnetic attraction between the magnet and the ferromagnetic additive will thus cause the compound to flow down into the gingival pocket 14 as compound exits the syringe. The compound material will begin to overflow the gingival edge when the pockets are filled. In this embodiment, it would be possible to either use a hand-held magnet as described above, or to use another type of fixture comprising a magnet attached to the teeth or around the jaw.

The above described method can also be used in conjunction with prior art methods for widening the sulcus, and can be used in a two-step process where a non magnetic compound is utilized for the remainder of the impression after the magnetic compound has been used to fill the gingival pockets.

### examples

The invention will be further illustrated by the following examples.

President ® impression compound was mixed with Carbonyl Iron powder from BASF having an average particle size of 1 µm. The Powder represented 23% of the finished mixture.

The elasticity of the 23% mixture was tested in accordance with the guidelines of the ISO 4823:2000(E) standard. Specimens of the 23% mixture were compressed 4 millimeters in 1 second and gradually released during 5 seconds. Their lengths were measured after 70 seconds of elastic recovery. The constant (K) was derived, with a K-value over 96.5 being regarded as acceptable. In the samples tested, the K-value of the 23% mixture was shown to be 99.599 compared to 99.78 for pure compound.

The ability of the 23% iron compound to reproduce detail was also tested. A standard test block with three lines carved into it as described in ISO 4823:2000(E) was used. By pouring the compound over the block an impression of the carved lines was formed. If the material was to be regarded as acceptable, all three lines should be visible with the use of a microscope. Actual observation confirmed a positive result.

In order to test the improved ability of the compound to penetrate into the sub-gingival areas, a clinically-relevant model of gingival pockets was constructed by stretching an elastic membrane over the outside of a glass beaker. Copper wires having 1 mm diameter were placed vertically between the membrane and the outside surface of the beaker, and arranged in intervals around the circumference of the vessel. The copper wires thus displaced the membrane from the surface of the glass, creating pockets mimicking gingival pockets ranging from 1mm to zero in width. Using a force meter the elasticity of the artificial gingival "tissue" was measured to be a clinically-relevant 10g per millimeter in the horizontal plane. By placing the model on digital scales and then penetrating the pockets with a periodontal probe the vertical force needed for penetration was measured as a clinically-relevant force of over 40g.

Six samples of the impression compound/iron mixture was applied to the gingival edge of the model. Then during 15 seconds a Neodymium Iron boron magnet, approximately 1 cm long and 0.5 cm in diameter, attached to a handle was moved vertically from the edge downwards ca. 2cm while contacting the compound, guiding the compound downwards into the pockets. The motion continued downwards and away from the model in a circular fashion. The same procedure was performed on six control samples of President ® impression compound without the added iron powder. The amount of penetration of the compound into the artificial gingival pockets was recorded for both the test samples and the control samples by measuring the distance from the gingival edge to the deepest penetration into the gingival pocket. The examples of the compound containing iron showed an average of five times greater degree of penetration than the pure compound.

## Claims

1. A dental impression material comprising an elastomeric dental impression compound to which is added a ferromagnetic compound.

2. The material of claim 1, wherein the compound is an additive comprising iron particles.

3. A material according to claim 2, wherein the average particle size is in the range of 0.3 µm - 20 µm

4. A material according to claim 2, wherein the average particle size is in the range of 0.5 µm- 5 µm

5. A material according to claim 2, wherein the average particle size is in the range of 0.8 µm - 2 µm.

6. A material according to any of claims 1-5, wherein the ferromagnetic compound represents from 10%-75% by weight of the compound.

7. A material according to any of claims 1-5, wherein the ferromagnetic compound represents from 15-55% by weight of the compound.

8. A material according to any of claims 1-5, wherein the ferromagnetic compound represents from 20%-30% by weight of the compound.

9. A material according to any of claims 1-8, wherein the elastomeric impression compound is a light body/flowing addition polymerized silicone impression material.

10. A method for guiding a flowable, elastomeric impression compound, comprising:
a. adding a ferromagnetic compound to the impression compound
b. guiding the compound by using a magnetic field to attract the ferromagnetic particles, thereby physically moving the impression compound.

11. A method according to claim 10 wherein the magnetic field is provided by a magnet.

12. A method according to claim 10 wherein the magnetic field has a Tesla value of less than 2.

## Patentansprüche

1. Zahnabdruckmaterial,
das eine elastomere Zahnabdruckverbindung aufweist, der eine ferromagnetische Verbindung zugesetzt ist.

2. Material nach Anspruch 1,
wobei die Verbindung ein Zusatzstoff ist, der Eisenpartikel aufweist.

3. Material nach Anspruch 2,
wobei die mittlere Partikelgröße im Bereich von 0,3 bis 20 µm liegt.

4. Material nach Anspruch 2,
wobei die mittlere Partikelgröße im Bereich von 0,5 bis 5 µm liegt.

5. Material nach Anspruch 2,
wobei die mittlere Partikelgröße im Bereich von 0,8 bis 2 µm liegt.

6. Material nach einem der Ansprüche 1 bis 5,
wobei die ferromagnetische Verbindung 10 bis 75 Gew.-% der Verbindung ausmacht.

7. Material nach einem der Ansprüche 1 bis 5,
wobei die ferromagnetische Verbindung 15 bis 55 Gew.-% der Verbindung ausmacht.

8. Material nach einem der Ansprüche 1 bis 5,
wobei die ferromagnetische Verbindung 20 bis 30 Gew.-% der Verbindung ausmacht.

9. Material nach einem der Ansprüche 1 bis 8,
wobei die elastomere Abdruckverbindung ein niedrigviskoses/fließfähiges, durch Additionspolymerisation erzeugtes Siliconabdruckmaterial ist.

10. Verfahren zum Führen einer fließfähigen elastomeren Abdruckverbindung, das folgendes aufweist:
a. Zugeben einer ferromagnetischen Verbindung zu einer Abdruckverbindung
b. Führen der Verbindung unter Verwendung eines Magnetfeldes, um ferromagnetische Partikel anzuziehen, wodurch sich die Abdruckverbindung körperlich bewegt.

11. Verfahren nach Anspruch 10,
wobei das Magnetfeld von einem Magneten bereitgestellt wird.

12. Verfahren nach Anspruch 10,
wobei das Magnetfeld einen Tesla-Wert von weniger als 2 aufweist.

## Revendications

1. Matériau d'empreinte dentaire comprenant un composé d'empreinte dentaire élastomérique auquel est ajouté un composé ferromagnétique.

2. Matériau selon la revendication 1, dans lequel le composé est un additif comprenant des particules de fer.

3. Matériau selon la revendication 2, dans lequel la dimension moyenne des particules se situe dans la plage de 0 , 3 µm à 20 µm .

4. Matériau selon la revendication 2, dans lequel la dimension moyenne des particules se situe dans la plage de 0,5 µm à 5 µm.

5. Matériau selon la revendication 2, dans lequel la dimension moyenne des particules se situe dans la plage de 0,8 µm à 2 µm.

6. Matériau selon l'une quelconque des revendications 1 à 5, dans lequel le composé ferromagnétique représente de 10 % à 75 % en poids du composé.

7. Matériau selon l'une quelconque des revendications 1 à 5, dans lequel le composé ferromagnétique représente de 15 % à 55 % en poids du composé.

8. Matériau selon l'une quelconque des revendications 1 à 5, dans lequel le composé ferromagnétique représente de 20 % à 30 % en poids du composé.

9. Matériau selon l'une quelconque des revendications 1 à 8, dans lequel le composé d' empreinte élastomérique est un matériau d'empreinte en silicone polymérisé d'addition à basse viscosité/fluide.

10. Procédé pour guider un composé d'empreinte élastomérique fluidifiable, consistant à :
a. ajouter un composé ferromagnétique au composé d' empreinte
b. guider le composé en utilisant un champ magnétique pour attirer les particules ferromagnétiques, afin de déplacer physiquement le composé d'empreinte.

11. Procédé selon la revendication 10, dans lequel le champ magnétique est procuré par un aimant.

12. Procédé selon la revendication 10, dans lequel le champ magnétique a une valeur Tesla inférieure à 2.
